# FASCICULE DE BREVET EUROPEEN

(11) **EP 0 509 919 B1**
(45) Date de publication et mention de la délivrance du brevet: **13.12.1995**
(21) Numéro de dépôt: 92401058.0
(22) Date de dépôt: 15.04.1992
(51) Int. Cl.: G01G 17/06, G21F 7/06, G01N 1/10

(54) **Installation automatisée de transfert et de pesage de cruchons contenant un liquide radioactif entre une unité de prélèvement et une chaîne d'analyse**
Automatische Anlage zum Fördern und Wiegen von mit einer radioaktiven Flüssigkeit gefüllten Flaschen zwischen einer Entnahmevorrichtung und einer Analysevorrichtung
Automatic system for the transfer and weighing of bottles filled with a radioactive liquid between a sampling unit and an analyser

(30) Priorité: 17.04.1991 FR 9104739
(43) Date de publication de la demande: 21.10.1992
(73) Titulaire: COGEMA COMPAGNIE GENERALE DES MATIERES NUCLEAIRES, F-78141 Velizy-Villacoublay (FR)
(72) Inventeur: Besnier, Joseph, F-50440 Beaumont Hague (FR); Gey, Jean François, F-50120 Equeurdreville (FR); Ferron, Denis, F-50120 Equeurdreville (FR)
(74) Mandataire: Poulin, Gérard

(56) Documents cités:
- EP-A- 0 078 212
- EP-A- 0 311 229
- EP-A- 0 364 078
- CH-A- 661 120
- GB-A- 2 200 469
- US-A- 4 470 265

## Description

L'invention concerne une installation automatisée conçue pour assurer le transfert de cruchons entre une ou plusieurs unités de prélèvement assurant l'introduction automatique d'un échantillon de liquide radioctif dans chaque cruchon et une ou plusieurs chaînes d'analyse, entourées d'une enceinte de protection biologique, cette installation permettant en outre de peser les cruchons lors de leur transfert.

Dans les usines de traitement de combustibles nucléaires, il est souhaitable de pouvoir prélever en différents points des échantillons des liquides radioactifs. Les échantillons prélevés permettent d'effectuer des analyses dont les résultats permettent notamment d'intervenir sur le traitement en cours pour en modifier certains paramètres.

Dans la pratique et comme cela est décrit notamment dans le document FR-A-2 515 350 (EP-A-78212), les prélèvements sont effectués dans des cruchons en matière plastique, qui sont placés dans des récipients appelés curseurs dont la forme particulière favorise le transfert des cruchons dans des tubes, sous l'action de moyens de transfert pneumatiques. Chaque cruchon est obturé par un opercule et préalablement mis sous vide.

Ce document montre également que les prélèvements sont effectués dans des unités de prélèvement à l'intérieur desquelles l'opercule obturant chacun des cruchons est piqué sur une aiguille de prélèvement dont l'extrémité opposée est reliée par un circuit approprié au point de l'usine auquel on désire effectuer le prélèvement. Plus précisément, chaque unité de prélèvement comporte plusieurs aiguilles communiquant par des circuits indépendants avec des parties différentes de l'usine, de telle sorte que plusieurs échantillons de liquides radioactifs différents peuvent être prélevés successivement sur une même unité de prélèvement. Le prélèvement proprement dit est réalisé par aspiration du liquide au travers de l'aiguille, sous l'effet de la dépression qui règne initialement dans le cruchon.

Dans une installation de ce type, les cruchons contenant les échantillons prélevés sont transférés pneumatiquement vers une ou plusieurs chaînes d'analyse, dans des enceintes confinées. Le tube servant au transfert des cruchons traverse chacune des enceintes selon une direction sensiblement verticale, de telle sorte que les cruchons chutent par gravité dans un réceptacle situé à l'intérieur de l'enceinte. Ce réceptacle comporte une bague coulissante qui isole le circuit pneumatique de l'intérieur de l'enceinte tant qu'un cruchon n'est pas admis dans cette dernière.

Lorsqu'une telle installation est automatisée, il peut se produire qu'un cruchon vide ou contenant une quantité de liquide insuffisante pour effectuer une analyse soit transféré à la chaîne d'analyse. Le liquide radioactif qui aurait dû se trouver dans le cruchon ne peut alors pas être analysé. Il est alors nécessaire, pour faire un nouveau prélèvement, de faire une nouvelle demande d'analyse, et le délai de réponse devient trop long. Par conséquent, il est souhaitable qu'un contrôle de l'état de remplissage de chacun des cruchons qui sont transférés des unités de prélèvement aux chaînes d'analyse puisse être effectué, afin par exemple qu'un nouveau prélèvement correspondant au prélèvement manquant puisse être effectué dans les meilleurs délais.

Par ailleurs, lorsque les analyses ont été effectuées, tout le liquide radioactif prélevé et tous les cruchons ayant servi aux prélèvements constituent des déchets qu'il est nécessaire de traiter selon les procédures habituelles de traitement des déchets radioactifs. Pour cette raison, il est souhaitable de limiter autant que possible la quantité de liquide radioactif prélevée ainsi que le nombre de cruchons utilisés. Cela impose de pouvoir contrôler le volume de chacun des échantillons prélevés afin que ce volume corresponde précisément à la ou aux analyses qui doivent être faites sur ce liquide. Cela impose également d'utiliser de préférence un seul cruchon lorsque le volume de liquide prélevé nécessaire à plusieurs analyses peut être contenu dans ce cruchon.

On connaît aussi du document GB-A-2 200 469 un système automatique, piloté par une unité centrale de commande, permettant d'effectuer successivement l'échantillonnage, la dilution et la pesée de liquides radioactifs à l'intérieur d'une boîte à gants.

L'invention a principalement pour objet une installation automatisée de transfert de cruchons dont la conception originale lui permette à la fois de détecter la présence d'un cruchon vide ou insuffisamment rempli de liquide radioactif et d'autoriser un contrôle plus rigoureux des échantillons de liquide prélevés conduisant à une réduction des déchets que constituent les liquides et les cruchons lorsque les analyses sont terminées.

Par ailleurs, il est important de noter qu'un tel résultat doit autant que possible pouvoir être obtenu sans accroître le temps de transfert des cruchons depuis chaque unité de prélèvement jusqu'aux chaîne d'analyse, et sans que les modifications apportées à l'installation de transfert ne nécessitent la mise en place d'une protection biologique supplémentaire coûteuse et entraînant un risque d'irradiation du personnel en cas d'intervention conduisant à enlever cette protection biologique, par suite d'une défaillance du système.

L'invention a donc également pour objet une installation automatisée de transfert de cruchons dans laquelle les opérations de contrôle effectuées sur chacun des cruchons sont réalisées pratiquement sans accroître le temps de transfert des cruchons et sans nécessiter de protection biologique supplémentaire pouvant entraîner un surcoût et des risques d'irradiation du personnel en cas d'intervention.

Enfin, l'invention a également pour objet une installation de transfert des cruchons dans laquelle ces différents résultats sont obtenus de façon totalement automatisée, afin de limiter autant que possible le nombre d'opérateurs et, par conséquent, de ne pas accroître le temps de transfert.

Conformément à l'invention, ces différents résultats sont atteints au moyen d'une installation automatisée de transfert de cruchons, entre au moins une unité de prélèvement assurant l'introduction automatique d'un échantillon de liquide radioactif dans chaque cruchon, et au moins une chaîne d'analyse entourée d'une enceinte confinée, cette installation comprenant des tubes reliant chaque unité de traitement à chacune des chaînes d'analyse et des moyens de transfert pneumatiques des cruchons dans ces tubes, caractérisée par le fait que les tubes débouchent dans un réceptacle placé à l'intérieur de l'enceinte de chaque chaîne d'analyse, l'installation comprenent de plus une unité centrale commandant les unités de prélèvement et les moyens de transfert pneumatiques, et des moyens de pesée des cruchons, associés à chacun des réceptacles.

Dans une installation ainsi définie, les moyens de pesée peuvent permettre de contrôler l'état de vacuité de certains des cruchons. Pour cela, ces moyens de pesée sont associés avec des moyens comparateurs qui comparent un signal de mesure délivré par les moyens de pesée à un seuil déterminé, puis adressent un signal de vacuité à l'unité centrale lorsque le signal de mesure est inférieur à ce seuil. Avantageusement, l'unité centrale commande alors un nouveau prélèvement analogue au prélèvement manquant, lors de la réception d'un tel signal de vacuité.

Par ailleurs, les informations fournies par les moyens de pesée peuvent également être utilisées directement afin de calculer le volume de liquide contenu dans chacun des cruchons, la densité de ce liquide étant par ailleurs connue pour chacun des points de prélèvement considérés. Une gestion plus rigoureuse des prélèvements peut alors être envisagée, ce qui conduit à réduire de façon appréciable la quantité de liquide rejetée après analyse, ainsi que la quantité de déchets solides que constituent les cruchons.

Par ailleurs, il est à noter que l'implantation des moyens de pesée sur les réceptacles qui sont placés à l'intérieur des enceintes de protection biologique de chaque chaîne d'analyse permet de ne pas avoir à ajouter de protection biologique supplémentaire. Le surcoût entraîné par un tel ajout est donc évité. De plus, une intervention peut éventuellement être effectuée, en cas de défaillance, directement dans la chaîne d'analyse, à l'aide des moyens de manutention à distance qui équipent habituellement celui-ci. Une telle intervention n'entraîne donc pratiquement aucun risque d'irradiation du personnel.

En outre, l'implantation des moyens de pesée sur les réceptacles des chaînes d'analyse permet, en ajoutant à l'installation des moyens d'éjection automatiques des cruchons après leur pesée, de n'entraîner pratiquement aucun allongement du temps de transfert des cruchons. Dans la pratique, ce moyen d'éjection peut être constitué par une buse d'éjection montée sur chacun des réceptacles et reliée à une source d'air comprimé commandée par l'unité centrale.

Selon un premier mode de réalisation de l'invention, les moyens de pesée comprennent un capteur de force monté à l'extrémité inférieure d'une bague coulissante, d'axe sensiblement vertical, constituant le réceptacle.

Selon un deuxième mode de réalisation de l'invention, les moyens de pesée comprennent un capteur de force monté sur un support fixe, sous un entonnoir solidaire d'une bague coulissante, d'axe sensiblement vertical, constituant le réceptacle, l'entonnoir étant décalé latéralement vers le bas par rapport à la bague coulissante, cette dernière comportant une rampe inférieure orientée vers ledit entonnoir.

Dans chacun de ces modes de réalisation, le capteur de force comprend avantageusement une partie inférieure de référence et une partie supérieure mobile verticalement par rapport à la partie inférieure et apte à venir en appui contre des surfaces de butée haute et basse de la partie inférieure lorsqu'une force excédant un seuil de tolérance des capteurs est exercée respectivement vers le haut ou vers le bas sur la partie supérieure. Cette caractéristique permet au capteur de force de supporter les chocs qui peuvent être produits par les cruchons remplis arrivant à grande vitesse et chutant par gravité.

Selon une disposition propre à l'industrie nucléaire, un ensemble électronique de mesure associé au capteur de force est placé à l'extérieur de l'enceinte confinée de la chaîne d'analyse et relié au capteur par un câble traversant cette enceinte.

Dans le cas où les cruchons sont réalisés en un matériau apte à se charger d'électricité statique tel qu'une matière plastique, la décharge de cette électricité statique sur le plateau des moyens de pesée servant à recevoir les cruchons pourrait entraîner une mesure erronée. Pour remédier à cet inconvénient, il est possible soit de réaliser ce plateau, au moins en surface, en un matériau électriquement isolant, soit de prévoir des moyens de décharge de l'électricité statique véhiculée par les cruchons dans le réceptacle, au-dessus des moyens de pesée.

On décrira maintenant, à titre d'exemple non limitatif, deux modes de réalisation préférés de l'invention, en se référant aux dessins annexés, dans lesquels :
- la figure 1 représente de façon très schématique une installation de transfert de cruchons conforme à l'invention ;
- la figure 2 est une vue en coupe partielle illustrant à plus grande échelle le réceptacle qui est monté dans l'enceinte de chaque chaîne d'analyse pour recevoir les cruchons contenant les échantillons prélevés, ainsi que le capteur de force associé à ce réceptacle afin d'assurer la pesée de chaque cruchon conformément à un premier mode de réalisation de l'invention ; et
- la figure 3 est une vue en coupe comparable à la figure 2, illustrant schématiquement un deuxième mode de réalisation de l'invention.

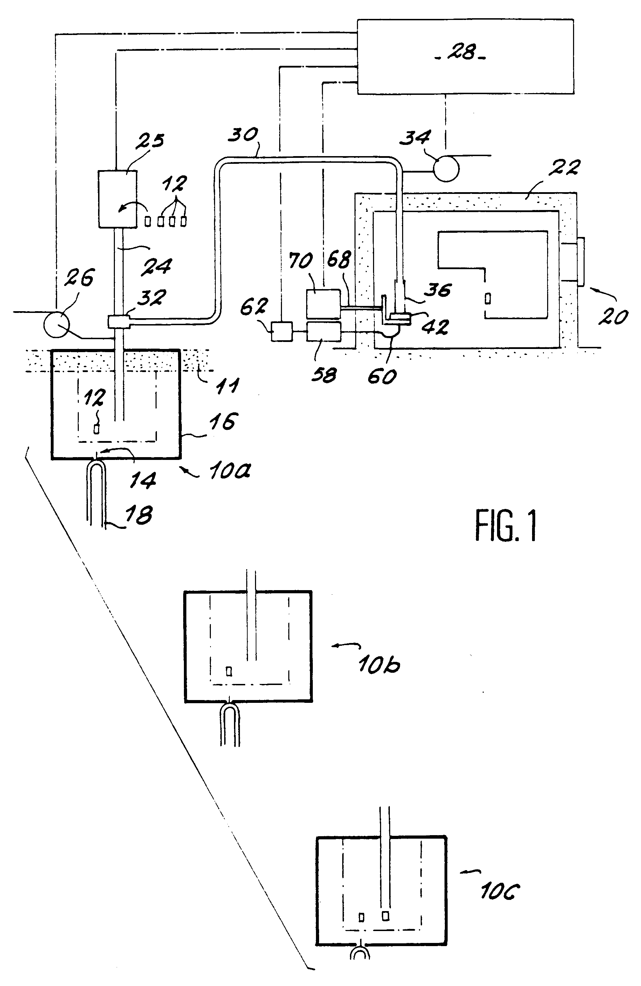

Sur la figure 1, les références 10a, 10b et 10c désignent de façon générale des unités de prélèvement dans lesquelles des cruchons 12, initialement sous vide, sont remplis à tour de rôle et de façon automatique d'échantillons de liquides radioactifs provenant de différents points d'une usine de traitement de combustibles nucléaires.

Chacune des unités de prélèvement 10a, 10b, 10c, dont le nombre peut varier selon le nombre et la répartition des points de l'usine dans lesquels un prélèvement doit être effectué, peut notamment être réalisée de la manière décrite dans le document FR-A-2 515 350.

Pour la bonne compréhension de l'invention, on indiquera simplement ici que les unités de prélèvement 10a, 10b et 10c disposent de moyens de manutention (non représentés), commandés à distance, grâce auxquels un cruchon sous vide 12 traverse une dalle 11 délimitant la zone contaminée, pour pénétrer dans une cuve 16 de l'unité de prélèvement. Les moyens de manutention positionnent ensuite le cruchon au-dessus d'une aiguille 14 choisie, selon un programme préétabli en fonction des analyses à effectuer, parmi un certain nombre d'aiguilles faisant saillie dans le fond de la cuve 16, puis piquent le cruchon sur cette aiguille. Un échantillon de liquide est alors automatiquement aspiré dans le cruchon sous l'effet de la dépression régnant initialement à l'intérieur de celui-ci. En effet, l'extrémité inférieure de l'aiguille 14 baigne alors dans une capacité dans laquelle le liquide radioactif à prélever est amené par un circuit 18, branché en dérivation sur le point de l'usine de traitement auquel on désire effectuer un prélèvement. Un exemple de réalisation du circuit 18 est décrit dans le document FR-A-2 516 242.

Les cruchons 12 dans lesquels sont effectués les prélèvements peuvent notamment être réalisés de la manière décrite dans le document FR-A-2 515 350. Dans ce cas, chaque cruchon comprend un flacon en matière plastique, obturé par un opercule en élastomère, de façon à former le cruchon proprement dit, et une enveloppe extérieure, également en matière plastique, appelée curseur, dont le profil est conçu de façon à permettre un transfert pneumatique des cruchons dans des tubes. Le cruchon se présente dans l'unité de prélèvement avec son opercule orienté vers le bas, de telle sorte que c'est cet opercule qui est piqué sur l'aiguille 14.

En variante, chacun des cruchons 12 peut aussi être réalisé sans enveloppe extérieure, le flacon présentant alors une forme extérieure identique à celle du curseur dans le cas précédent.

Sur la figure 1, on a également représenté de façon schématique l'installation permettant de transférer les cruchons vides jusqu'à chacune des unités de prélèvement 10a, 10b et 10c, puis de transférer les cruchons contenant les échantillons jusqu'à un ou plusieurs chaînes d'analyse 20, une seul de ces chaînes ayant été illustrée sur la figure pour en faciliter la lecture. Chaque chaîne d'analyse 20 est délimitée par une enceinte confinée de protection biologique 22.

L'installation de transfert des cruchons comprend tout d'abord des tubes 24 dans lesquels les cruchons sous vide 12 sont transférés depuis un ou plusieurs postes 25 de distribution des cruchons 12 sous vide jusqu'à chacune des unités de prélèvement 10a, 10b et 10c. Le transfert successif de chacun des cruchons 12 dans les tubes 24 est assuré par des moyens pneumatiques qui comprennent une pompe d'aspiration 26 branchée en dérivation à l'entrée de chacune des unités de prélèvement. Cette pompe 26 est pilotée, de même que les moyens de manutention des cruchons 12 associés à chacune des unités de prélèvement et que les postes 25 de distribution des cruchons, par une unité centrale de commande 28.

L'installation de transfert de cruchons selon l'invention comprend de plus des tubes 28 qui relient chacune des unités de prélèvement 10a, 10b et 10c à chacune des chaînes d'analyse 20. Les tubes 30 sont équipés d'aiguillages (non représentés), également pilotés par l'unité centrale de commande 28, qui permettent de diriger à volonté et de façon automatisée chacun des cruchons 12 provenant de l'une quelconque des unités de prélèvement 10a, 10b et 10c vers l'une ou l'autre des chaînes d'analyse, afin que les analyses requises puissent être effectuées sur l'échantillon prélevé.

Si la sortie des cruchons hors des unités de prélèvement s'effectue par le même chemin que leur entrée, des aiguillages 32, également pilotés par l'unité centrale de commande 28, sont placés entre les tubes 24 et 30 comme l'illustre la figure 1.

Le transfert des cruchons 12 contenant les échantillons pré levés depuis les unités de prélèvement jusque dans les chaînes d'analyse s'effectue grâce à des moyens pneumatiques comprenant par exemple des pompes à dépression 34, pilotées par l'unité centrale de commande 28 et branchées en dérivation sur les tubes 30, à l'entrée de chacune des chaînes d'analyse 20.

La portion de chacun des tubes de transfert 30 qui pénètre dans les chaînes d'analyse 20 en traversant l'enceinte de protection biologique 22, est orientée selon une direction sensiblement verticale, de telle sorte que les cruchons 12 chutent dans un réceptacle comprenant une bague coulissante 36. La bague coulissante 36 occupe normalement une position haute, dans laquelle elle obture la communication entre le tube 30 et l'intérieur de l'enceinte de protection biologique 22, ce qui permet le déplacement des cruchons dans le tube 30 sous l'action de la pompe 34 et préserve le confinement de l'atmosphère en dépression contenue à l'intérieur de l'enceinte de protection biologique 22.

Dès qu'un capteur (non représenté) détecte l'arrivée d'un cruchon sur le réceptacle 36 occupant cette position haute, une descente de la bague coulissante à l'intérieur de l'enceinte de protection biologique 22 est commandée depuis l'unité centrale de commande 28. Cela peut notamment être obtenu à l'aide d'une buse (non représentée) orientée selon une direction horizontale et reliée à une source d'air comprimé, de façon à injecter de l'air comprimé au-dessus du réceptacle 36 contenant le cruchon 12.

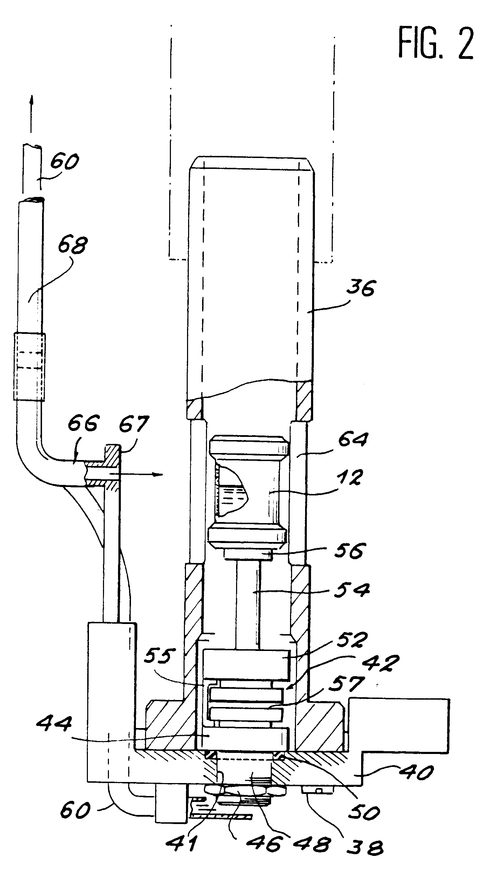

Dans un premier mode de réalisation de l'invention illustré plus en détail la figure 2, l'extrémité inférieure de la bague coulissante 36 est fixée, par exemple au moyen de vis 38, sur une plaque support horizontale 40. Cette plaque support 40 est percée, dans le prolongement de la bague coulissante 36, d'un trou 41 servant à fixer au-dessus de la plaque 40 un capteur de force désigné de façon générale par la référence 42.

De façon plus précise, le capteur de force 42 comporte une partie inférieure 44 qui est fixée au-dessus de la plaque support 40. A cet effet, un écrou 46 est vissé sur une tige filetée 48 appartenant à la partie inférieure 44 et traversant le trou 41 formé dans la plaque support 40. L'étanchéité entre la partie inférieure 44 du capteur de force 42 et la plaque support 40 est assurée par un joint torique 50, emprisonné entre les deux pièces.

Le capteur de force 42 comporte également une partie supérieure 52, mobile selon l'axe sensiblement vertical du capteur à l'intérieur de la partie basse de la bague coulissante 36. Cette partie supérieure 52 du capteur de force 42 est prolongée vers le haut selon l'axe de la bague coulissante 36 par une tige 54 portant un plateau 56 à son extrémité supérieure.

Des jauges de contrainte (non représentées sur la figure 2) sont interposées entre la partie supérieure 52 et la partie inférieure 44 du capteur de force 42, de telle sorte que, lorsqu'un cruchon 12 repose sur le plateau 56, un signal électrique de mesure représentatif du poids total du cruchon 12 est émis par ces jauges de contrainte. Ce signal est transmis à un ensemble électronique de mesure 58 (figure 1) situé à l'extérieur de l'enceinte de protection biologique 22 par un câble 60.

Lorsque les moyens de pesée des cruchons, que constituent le capteur de force 42 et l'ensemble électronique de mesure 58 qui lui est associé, ont pour fonction essentielle de détecter l'arrivée d'un cruchon vide ou insuffisamment rempli à l'une quelconque des chaînes d'analyse 20, le signal de mesure sortant de l'ensemble électronique de mesure 58 est transmis à des moyens comparateurs 62 (figure 1). A l'intérieur de ces moyens comparateurs, le signal de mesure est comparé à un seuil déterminé en dessous duquel on estime que le cruchon 12 est vide ou insuffisamment rempli. Si tel est le cas, un signal de vacuité est émis et transmis par les moyens comparateurs 62 à l'unité centrale de commande 28. Cette unité peut alors adresser les ordres nécessaires aux différents organes de l'installation qu'elle commande, afin qu'un nouveau prélèvement analogue au prélèvement qui aurait dû se trouver dans le cruchon vide ou insuffisamment rempli soit effectué et adressé à nouveau à la chaîne d'analyse correspondant.

Dans une variante de réalisation non représentée, les signaux de mesure délivrés par l'ensemble électronique de mesure 58 peuvent être transmis directement à l'unité centrale de commande 28, afin de permettre un pilotage quantitatif de chacun des prélèvements effectués par l'installation, pour que les quantités de liquide radioactif juste nécessaires aux analyses à effectuer soient prélevées. L'échantillon contenu dans chacun des cruchons peut alors servir, au moins dans certains cas, à effectuer plusieurs analyses différentes du même liquide radioactif. La mesure du poids de chacun des cruchons effectuée par les moyens de pesée comprenant le capteur de force 42 et l'ensemble électronique de mesure associés 58 permet en effet de calculer les volumes prélevés, étant donné que la densité des liquides radioactifs prélevés est par ailleurs connue. Cette variante de réalisation permet d'optimiser la gestion des déchets produits par l'installation, puisque les quantités de liquide radioactif et de cruchons qui sont rejetées après analyse peuvent ainsi être minimisées.

En se reportant à nouveau à la figure 2, on voit que la partie supérieure 52 du capteur de force 42 peut se déplacer selon une direction sensiblement verticale par rapport à la partie inférieure 44 entre une butée haute 55 de traction et une butée basse 57 de compression formées sur cette dernière partie et limitant ce déplacement dans l'un et l'autre sens. Cette caractéristique permet d'éviter que le choc produit par l'arrivée d'un cruchon 12 à grande vitesse sur le plateau 56 n'endommage le capteur.

On voit également sur la figure 2 que, lorsqu'un cruchon 12 repose sur le plateau 56 à l'intérieur de la bague coulissante 36, il se trouve en face d'une fenêtre 64 formée dans cette dernière. Par ailleurs, une buse 66, reliée par un support 67 à la plaque support 44, débouche selon une direction sensiblement horizontale d'un côté de la fenêtre 64. Cette buse 66 est reliée par un tuyau souple 68 à une source d'air comprimé 70 (figure 1) située à l'extérieur de l'enceinte de protection biologique 22.

La source d'air comprimé 70 est pilotée par l'unité centrale de commande 28, de telle sorte que de l'air comprimé s'échappe de la buse d'éjection 66 dès que la pesée du cruchon 12, à l'aide des moyens de pesée décrits précédemment, a été effectuée. L'air comprimé sortant de la buse 66 a pour effet d'éjecter le cruchon 12 au travers de la fenêtre 64, pour le faire tomber sur un appareil (non représenté), tel qu'une rampe ou un transporteur, permettant de l'acheminer jusqu'au poste d'analyse concerné de la chaîne 20.

Enfin, étant donné que les cruchons 12 sont le plus souvent réalisés en matière plastique qui risque de se charger d'électricité statique lors de son transfert à l'intérieur des tubes 30, des précautions peuvent avantageusement être prises afin que cette électricité statique ne soit pas déchargée sur le capteur de force 42, ce qui risquerait d'en fausser les mesures.

Pour cela, une première solution consiste à réaliser le capteur 42, au moins dans la partie du plateau 56 sur laquelle vient se poser le cruchon 12, en un matériau non conducteur de l'électricité.

Une autre solution consiste à prévoir dans le réceptacle 36 ou dans la partie attenante du tube 30 des moyens de décharge de l'électricité statique dont peuvent être chargés les cruchons 12.

Dans un deuxième mode de réalisation de l'invention illustré sur la figure 2, le capteur de force 42 n'est pas lié à la bague coulissante 36, mais à une pièce fixe telle que la partie du tube de transfert 30 qui descend verticalement dans la chaîne d'analyse 20. Par ailleurs, le capteur de force 42 est décalé latéralement et vers le bas par rapport à la bague coulissante 36. Ces caractéristiques permettent de réduire très sensiblement les chocs subis par les capteurs lors de l'arrivée des cruchons et, par conséquent, de diminuer très nettement les risques de défaillance des capteurs dus à ces chocs.

De façon plus précise, le capteur de force 42 est monté verticalement dans un logement 72 formé dans un boîtier de protection 74. Ce boîtier 74 est relié à l'extrémité inférieure du tube de transfert 30 par une structure rigide 76. De plus, l'axe vertical du logement 72 est décalé latéralement par rapport à l'axe vertical de l'extrémité inférieure du tube 30.

Par ailleurs, la bague coulissante 36 comporte sur sa face supérieure une rampe inclinée 78, tournée vers le logement 72. Un entonnoir de guidage 80, solidarisé de la bague 36 par exemple par des vis 82, est placé au-dessus du logement 72, dans le prolongement de la rampe inclinée 78.

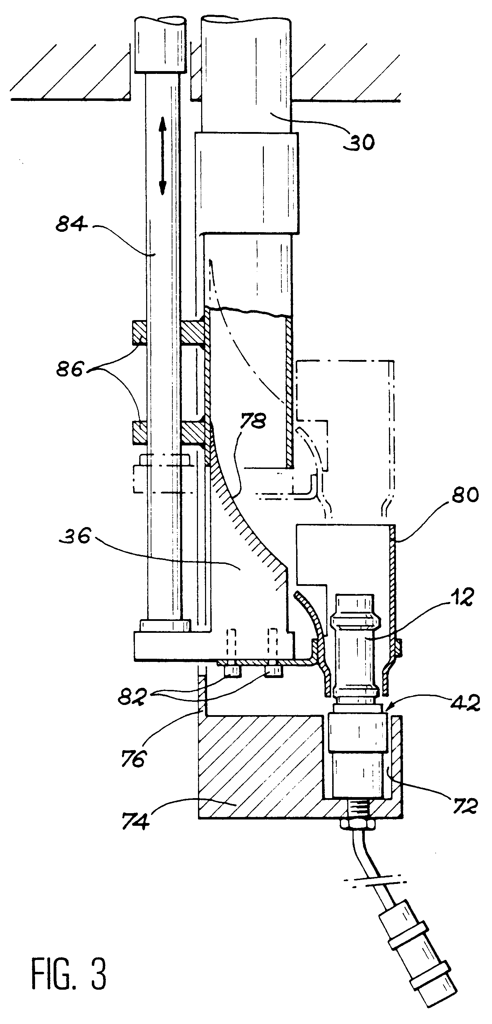

La bague coulissante 36 et l'entonnoir 80 constituent un ensemble mobile entre une position haute (illustrée en traits mixtes sur la figure 3) et une position basse illustrée en trait plein.

Dans la position haute de cet ensemble mobile, la rampe 78 ferme le tube 30 à son extrémité inférieure et l'entonnoir 80 est écarté du capteur de force 42 d'une distance au moins égale à la hauteur d'un cruchon 12. Une buse d'air comprimé (non représentée) analogue à la buse 66 sur la figure 2 et débouchant au-dessus du boîtier de protection 74 permet alors d'évacuer le cruchon 12 après sa pesée.

Dans la position basse de l'ensemble mobile, le cruchon 12 descend par gravité sur la rampe inclinée 78 et pénètre dans l'entonnoir 80, pour venir se poser sans choc sur le capteur de force 42.

Dans l'exemple de réalisation illustré sur la figure 3, le guidage de l'ensemble mobile est assuré par une colonnette verticale 84, solidaire de la bague coulissante 36 et reçue dans des oreilles de guidage 86, solidaires du tube 30.

Lorsqu'un cruchon 12 arrive dans la chaîne d'analyse 20, l'ensemble bague coulissante 36-entonnoir 80 est en position haute et ferme le tube 30. La descente de cet ensemble ouvre le tube 30 et permet la descente du cruchon, par gravité, jusqu'au capteur de force 42. Pendant que la pesée se réalise, l'ensemble bague coulissante 36-entonnoir 80 remonte. Le cruchon 12 peut ensuite être évacué par un jet d'air comprimé.

L'installation automatisée de transfert de cruchons qui vient d'être décrite en se référant aux figures 1 à 3 permet de peser les cruchons à leur arrivée dans chacune des chaîne d'analyse, ce qui autorise comme on l'a vu précédemment la réalisation immédiate d'un nouveau prélèvement dans le cas d'un prélèvement défectueux et/ou un contrôle plus rigoureux des déchets liquides et solides sortant des différentes chaînes d'analyse.

Par ailleurs, l'implantation particulière décrite des moyens de pesée permet de ne pas avoir à prévoir autour de ces moyens de pesée une protection neutronique supplémentaire et autorise une intervention sans risque en cas de défaillance du système.

Enfin, la cadence de l'installation n'est pratiquement pas ralentie par l'introduction des moyens de pesée, étant donné qu'il existe de toute manière entre deux cruchons consécutifs acheminés à l'intérieur des tubes une distance suffisante pour que le pesage et l'éjection de l'un d'entre eux soient effectués avant qu'un autre cruchon ne parvienne jusqu'aux moyens de pesage.

Bien entendu, l'invention n'est pas limitée au mode de réalisation qui vient d'être décrit à titre d'exemple, mais en couvre toutes les variantes. Ainsi, il est clair que l'installation de transfert selon l'invention peut comprendre un nombre quelconque d'unités de prélèvement et de chaînes d'analyse. De plus, ces unités de prélèvement et ces chaînes d'analyse peuvent être réalisés d'une manière quelconque, sans sortir du cadre de l'invention. De même, les postes de distribution des cruchons sous vide, les aiguillages prévus dans les tubes de transfert, ainsi que les moyens de transfert pneumatiques des cruchons à l'intérieur de ces tubes peuvent être réalisés d'une manière quelconque.

## Revendications

1. Installation automatisée de transfert de cruchons (12), entre au moins une unité de prélèvement (10a,10b,10c) assurant l'introduction automatique d'un échantillon de liquide radioactif dans chaque cruchon, et au moins une chaîne d'analyse (20) entourée d'une enceinte confinée (22), cette installation comprenant des tubes (30) reliant chaque unité de traitement (10a, 10b,10c) à chacune des chaînes d'analyse et des moyens de transfert pneumatiques (34) des cruchons (12) dans ces tubes, caractérisée par le fait que les tubes (30) débouchent dans un réceptacle (36) placé à l'intérieur de l'enceinte de chaque chaîne d'analyse, l'installation comprenant de plus une unité centrale (28) commandant les unités de prélèvement et les moyens de transfert pneumatiques, et des moyens de pesée (42,58) des cruchons, associés à chacun des réceptacles.

2. Installation selon la revendication 1, caractérisée par le fait que les moyens de pesée comprennent un capteur de force (42) monté à l'extrémité inférieure d'une bague coulissante (36), d'axe sensiblement vertical, constituant le réceptacle.

3. Installation selon la revendication 1, caractérisée par le fait que les moyens de pesée comprennent un capteur de force (42) monté sur un support fixe (74), sous un entonnoir (80) solidaire d'une bague coulissante (36), d'axe sensiblement vertical, constituant le réceptacle, l'entonnoir (80) étant décalé latéralement et vers le bas par rapport à la bague coulissante, cette dernière comportant une rampe inférieure (78) orientée vers ledit entonnoir.

4. Installation selon l'une quelconque des revendications 2 et 3, caractérisée par le fait que le capteur de force (42) comprend une partie inférieure de référence (44), et une partie supérieure (52) mobile verticalement par rapport à la partie inférieure (44), et apte à venir en appui contre des surfaces de butée haute et basse (55, 57) de la partie inférieure, lorsqu'une force excédant un seuil de tolérance du capteur est exercée respectivement vers le haut et vers le bas sur la partie supérieure.

5. Installation selon l'une quelconque des revendications 2 à 4, caractérisée par le fait que les moyens de pesée comprennent de plus un ensemble électronique de mesure (58), placé à l'extérieur de l'enceinte confinée (22) et relié au capteur de force (42) par un câble (60) traversant l'enceinte.

6. Installation selon l'une quelconque des revendications précédentes, caractérisée par le fait qu'une buse d'éjection (66) des cruchons est montée sur chacun des réceptacles (36) et reliée à une source d'air comprimé (70) commandée par l'unité centrale (28).

7. Installation selon l'une quelconque des revendications précédentes, caractérisée par le fait qu'elle comprend des moyens comparateurs (62) aptes à comparer un signal de mesure délivré par les moyens de pesée (42, 58) à un seuil déterminé, ces moyens comparateurs adressant un signal de vacuité à l'unité centrale (28) lorsque le signal de mesure est inférieur à ce seuil.

8. Installation selon la revendication 7, caractérisée par le fait que l'unité centrale (28) commande un nouveau prélèvement analogue au prélèvement manquant, lors de la réception d'un signal de vacuité.

9. Installation selon l'une quelconque des revendications précédentes, caractérisée par le fait que, les cruchons (12) étant réalisés en un matériau apte à se charger d'électricité statique, un plateau (56) des moyens de pesée (42) apte à recevoir les cruchons est réalisé, au moins en surface, en un matériau électriquement isolant.

10. Installation selon l'une quelconque des revendications 1 à 8, caractérisée par le fait que les cruchons (12) étant réalisés en un matériau apte à se charger d'électricité statique, des moyens de décharge de cette dernière sont prévus dans le réceptacle (36), au-dessus des moyens de pesée (42).

## Claims

1. Automated installation for the transfer of pots (12) between at least one sampling unit (10a,10b,10c) ensuring the automatic introduction of a radioactive liquid sample into each pot, and at least one analysis chain or line (20) surrounded by a confined enclosure (22), said installation comprising tubes (30) connecting each processing unit (10a,10b,10c) to each of the analysis chains and means for the pneumatic transfer (34) of the pots (12) into said tubes (30), characterized in that the tubes issue into a receptacle (36) placed within the enclosure of each analysis chain, the installation also comprising a central unit (28) controlling the sampling units and the pneumatic transfer means, and means (42,58) for weighing the pots associated with each of the receptacles.

2. Installation according to claim 1, characterized in that the weighing means comprise a force transducer (42) fitted at the lower end of a substantially vertically axed slide ring (36) constituting the receptacle.

3. Installation according to claim 1, characterized in that the weighing means comprise a force transducer (42) fitted to a fixed support (74), below a funnel (80) integral with a substantially vertically axed slide ring (36) constituting the receptacle, the funnel (80) being laterally and downwardly displaced with respect to the slide ring, the latter having a lower ramp (78) oriented towards the said funnel.

4. Installation according to either of the claims 2 and 3, characterized in that the force transducer (42) has a lower reference part (44) and an upper part (52) which is vertically mobile relative to the lower part (44) and which can bear against upper and lower abutment surfaces (55,57) of the lower part when a force exceeding a tolerance threshold of the transducer is respectively exerted upwards and downwards on the upper part.

5. Installation according to any one of the claims 2 to 4, characterized in that the weighing means also incorporate an electronic measuring means (58) positioned outside the confined enclosure (22) and connected to the force transducer (42) by a cable (60) traversing the enclosure.

6. Installation according to any one of the preceding claims, characterized in that the pot ejection nozzle (66) is fitted to each of the receptacles (36) and connected to a compressed air source (70) controlled by the central unit (28).

7. Installation according to any one of the preceding claims, characterized in that comparison means (62) are also provided, which are able to compare a measuring signal supplied by the weighing means (42,58) with a given threshold, said comparison means addressing an emptiness signal to the central unit (28) when the measuring signal is below said threshold.

8. Installation according to claim 7, characterized in that the central unit (28) controls a new sampling operation identical to the missing sampling operation, on receiving an emptiness signal.

9. Installation according to any one of the preceding claims, characterized in that the pots (12) are made from a material which can be charged with static electricity, so that a disk (56) of the weighing means (42) for receiving the pots is made, at least on its surface, from an electrically insulating material.

10. Installation according to any one of the claims 1 to 8, characterized in that the pots (12) are made from a material which can be charged with static electricity, so that means for discharging the latter are provided in the receptacle (36) above the weighing means (42).

## Patentansprüche

1. Automatische Anlage zum Fördern von Behältern (12) zwischen mindestens einer Entnahmeeinheit (10a, 10b, 10c), die das automatische Eingeben einer radioaktiven Flüssigkeitsprobe in jeden Behälter gewährleistet, und mindestens einer Analysekette (20), die von einer Sicherheitsumhüllung (22) umgeben ist, wobei diese Anlage Rohre (30) aufweist, die jede Behandlungseinheit (10a, 10b, 10c) mit jeder der Analyseketten verbinden, und Mittel zum pneumatischen Befördern (34) der Behälter (12) in den Rohren aufweist,
**dadurch gekennzeichnet**,
daß die Rohre (30) in eine Aufnahme (36) münden, die im Inneren der Umhüllung jeder Analysekette angeordnet ist, und die Anlage außerdem aufweist: eine Zentraleinheit (28), die die Entnahmeeinheiten und die Mittel zum pneumatischen Befördern steuert, und Wägemittel (42, 58) für die Behälter, jeweils einem der Aufnahmen zuordnet.

2. Anlage nach Anspruch 1,
**dadurch gekennzeichnet**,
daß die Wägemittel einen Kraftaufnehmer (42) aufweisen, der am unteren Ende eines Verschieberings (36) angeordnet ist, der mit etwa vertikaler Achse die Aufnahme bildet.

3. Anlage nach Anspruch 1,
**dadurch gekennzeichnet**,
daß die Wägemittel einen Kraftaufnehmer (42) aufweisen, der an einem ortsfesten Träger (74) unterhalb eines die Aufnahme bildenden Verschieberings mit genau vertikaler Achse gelagert ist, wobei der Trichter (80) seitlich und nach unten bezüglich des Verschieberings versetzt ist, wobei letzterer eine untere Rampe (78) aufweist, die zu dem Trichter hin orientiert ist.

4. Anlage nach einem der Ansprüche 2 und 3,
**dadurch gekennzeichnet**,
daß der Kraftaufnehmer (42) ein unteres Referenzteil (44) und ein oberes Teil (52) aufweist, das in Bezug auf das untere Teil (44) vertikal beweglich und dazu ausgebildet ist, in Anlage mit oberen und unteren Anschlagflächen (55, 57) des unteren Teils zu kommen, wenn eine eine Toleranzschwelle des Aufnehmers übersteigende Kraft nach oben bzw. nach unten gegen das obere Teil ausgeübt wird.

5. Anlage nach einem der Ansprüche 2 bis 4,
**dadurch gekennzeichne**t,
daß die Wägemittel außerdem eine elektronische Meßeinrichtung (58) aufweisen, die sich außerhalb der Sicherheitsumhüllung (22) befindet und mit dem Kraftaufnehmer (42) über eine die Umhüllung durchsetzende Leitung (60) verbunden ist.

6. Anlage nach einem der vorhergehenden Ansprüche,
**dadurch gekennzeichnet**,
daß an jeder der Aufnahmen (36) eine Ausstoßdüse (66) für die Behälter gelagert und mit einer von der Zentraleinheit (28) gesteuerten Druckluftquelle (70) verbunden ist.

7. Anlage nach einem der vorhergehenden Ansprüche,
**dadurch gekennzeichnet**,
daß sie Vergleichermittel (62) aufweist, mit denen ein von den Wägemitteln (42, 48) geliefertes Meßsignal mit einem bestimmten Schwellenwert verglichen wird, wobei diese Vergleichermittel an die Zentraleinheit (28) ein Leersignal geben, wenn das Meßsignal unterhalb dieses Schwellenwerts liegt.

8. Anlage nach Anspruch 7,
**dadurch gekennzeichnet**,
daß die Zentraleinheit (28) bei Erhalt eines Leersignals eine neue Entnahme analog zu der Fehl-Entnahme veranlaßt.

9. Anlage nach einem der vorhergehenden Ansprüche,
**dadurch gekennzeichnet,**
daß die Behälter (12) aus einem Material bestehen, welches sich möglicherweise elektrostatisch auflädt, und eine Platte (56) der Wägemittel (42) zur Aufnahme der Behälter zumindest an der Oberfläche aus einem elektrischen Isolierstoff gebildet ist.

10. Anlage nach einem der Ansprüche 1 bis 8,
**dadurch gekennzeichnet**,
daß die Behälter (12) aus einem Material bestehen, welches sich möglicherweise elektrostatisch auflädt, und das Entladungsmittel für letztere in der Aufnahme (36) oberhalb der Wägemittel (42) vorgesehen sind.
